(19) Europäisches Patentamt
European Patent Office
Office européen des brevets

(11) Veröffentlichungsnummer : **0 392 429 B1**

(12) # EUROPÄISCHE PATENTSCHRIFT

(45) Veröffentlichungstag der Patentschrift : **24.03.93 Patentblatt 93/12**

(51) Int. Cl.[5] : **A61K 31/365,** A61K 9/08, A61K 47/10

(21) Anmeldenummer : **90106786.8**

(22) Anmeldetag : **09.04.90**

(54) **Psoralen-Reagens.**

(30) Priorität : **11.04.89 US 336179**

(43) Veröffentlichungstag der Anmeldung : **17.10.90 Patentblatt 90/42**

(45) Bekanntmachung des Hinweises auf die Patenterteilung : **24.03.93 Patentblatt 93/12**

(84) Benannte Vertragsstaaten : **AT BE CH DE DK FR GB IT LI NL**

(56) Entgegenhaltungen :
**GB-A- 2 052 980**
**US-A- 4 321 919**
**CHEMICAL ABSTRACTS, Band 97, Nr. 2, Juli 1982, Seite 361, Spalte 1, Zusammenfassung Nr. 11747c, Columbus, Ohio, US; Y. XU: "Formulation studies on psoralen and isopsoralen injections"**

(73) Patentinhaber : **F. HOFFMANN-LA ROCHE AG**
**Postfach 3255**
**CH-4002 Basel (CH)**

(72) Erfinder : **Bachynsky, Maria Oksana**
**38 Carrie Court**
**Nutley, N.J. 07110 (US)**
Erfinder : **Infeld, Martin Howard**
**6 Tuers Place**
**Upper Montclair, N.J. 07043 (US)**
Erfinder : **Margolis, Richard Joel**
**29 Jeffry Road**
**Wayne, N.J. 07470 (US)**
Erfinder : **Perla, Dennis Anthony**
**15 Colborn Court**
**Wayne, N.J. 07470 (US)**

(74) Vertreter : **Lederer, Franz, Dr. et al**
**Lederer, Keller & Riederer, Patentanwälte,**
**Lucile-Grahn-Strasse 22**
**W-8000 München 80 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Psoralen ist ein linearer Dreiring-Heterocyclus mit der allgemeinen Struktur

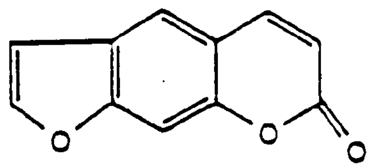

Es ist ein bifunktionelles photoreaktives Molekül, das mit Nukleinsäuren in Gegenwart von langwelligem ultraviolettem Licht kovalente Bindungen eingeht. Die Fähigkeit des Psoralens, mit DNA zu reagieren, hat ihm klinische Bedeutung bei der Behandlung verschiedener Hautkrankheiten verliehen, und seine Fähigkeit, Quervernetzungen zwischen den Strängen doppelsträngiger DNA herzustellen hat es zu einem wertvollen Mittel bei der Untersuchung von Struktur und Funktion von Nukleinsäuren werden lassen.

Seit mehr als 10 Jahren sind Psoriasis-Patienten mit oral verabreichtem Psoralen behandelt und anschliessend mit langwelligem UV-A-Licht (PUVA) behandelt worden. Gegenwärtig ist das einzige, kommerziell verfügbare Psoralen für orale Verabreichung 8-Methoxypsoralen (8-MOP). Da Psoralene lipophile, in Wasser schwer lösliche Verbindungen sind, besteht ein geläufiges orales Präparat aus 8-MOP, das mit Polyäthylenglykolen vermischt, in einer Weichgelatinekapsel enthalten ist. Die orale Resorption von 8-MOP aus dieser Dosierungsform ist individuell beträchtlich verschieden, so können Spitzenwerte von Serumpsoralen bereits 1 Stunde oder erst 4 Stunden nach Verabreichung der Kapsel eintreten. Die UV-A-Bestrahlung des Patienten erfolgt etwa 2 Stunden nach oraler Verabreichung der Dosierungsform. Da die orale Resorption von 8-MOP aus der Kapsel variabel ist, ist es möglich, dass 2 Stunden nach der Dosierung die Serumspiegel von 8-MOP nicht optimal sind. Da die 8-MOP-Serumspiegel und die Photosensitivität in direkter Korrelation stehen, können suboptimale 8-MOP-Serumspiegel ein Faktor für ein unzureichendes Ansprechen auf PUVA-Therapie sein.

Es sind auch andere Verabreichungsweisen für 8-MOP verwendet worden. In den Vereinigten Staaten ist 8-MOP als topische Lotion erhältlich. In Europa ist 8-MOP topisch mittels Bädern angewandt worden. PUVA-Bäder sind wirksam, aber unbequem, und die topische Resorption von 8-MOP ist unterschiedlich, was dazuführt, dass die phototoxische Reaktion nicht vorausgesagt werden kann. PUVA-Bäder haben daher keine weite Verbreitung gefunden.

Serumspiegel und Photosensitivität sind mit rektal verabreichtem 8-MOP besser voraussehbar und die Behandlung spricht therapeutisch gut an. Jedoch hat die Unbequemlichkeit der rektalen Verabreichung wahrscheinlich dazu geführt, dass sie keine weit verbreitete Popularität erlangt hat.

Andere orale Dosierungsformen sind Lösungen, in denen Psoralen in Alkoholen und/oder Polyäthylenglykolen gelöst sind oder als Oel-in-Wasser-Emulsionen vorliegen, oder sie stellen halb-feste Kapselformulierungen dar, in denen die mittlere Kristallgröße der Psoralene stark reduziert ist. Diese Dosierungsformen haben die PUVA-Therapie verbessert, aber die Unvorhersehbarkeit der oralen Verabreichung von Psoralenen bedingt immer noch ein ungleichmäßiges therapeutisches Ansprechen auf Photophorese-Therapie.

Es besteht daher ein Bedürfnis für eine Psoralen-Dosierungsform, die gleichmäßige Resultate liefert, wenn Psoralene in Verbindung mit UV-A-Phototherapie angewandt werden, um Krankheiten zu heilen.

Die vorliegende Erfindung betrifft ein Psoralen-Reagens, enthaltend eine sterile, wässrige Lösung einer kleinen wirksamen Menge an Psoralen, die zur Erreichung therapeutischer Blutspiegel ausreicht, Aethylalkohol, Propylenglykol und, gewünschtenfalls, ein Puffersystem.

Die vorliegende Erfindung betrifft weiterhin ein solches Reagens, das in einem braunen Glasbehälter aufbewahrt wird, der mit einem Gummistopfen versehen ist, der, mit einem Fluor-Harz laminiert (teflonisiert) ist.

Der hier verwendete Ausdruck "Photophorese" bezeichnet extrakorporale Phototherapie, bei der dem Patienten Blut entnommen wird, das in Leukozyten- und Leukozyten-arme Fraktionen aufgetrennt wird. Die Erythrozyten (Leukozyten-arme Fraktion) werden sogleich in den Patienten zurückgeführt und die Leukozytenreiche Fraktion wird der UV-Strahlung ausgesetzt und dann in den Patienten zurückgeleitet. Ultraviolett-A bezeichnet Licht des Ultraviolett-A-Spektrums von $3\,200 \cdot 10^{-10}$ m - $4\,000 \cdot 10^{-10}$ m (3'200-4'000 Angström). Leukozyten-reich bezeichnet die Leukozyten-reiche Fraktion aus Blut, aus dem die Erythrozyten und Bestandteile die nicht Leukozyten sind, entfernt worden sind.

Der Ausdruck "Leukozyten-mediierte Krankheiten" bezeichnet Krankheiten oder Zustände, bei denen die Manipulation der Leukozytenfunktion mit Psoralen und Phototherapie zu einer Behebung der Krankheit oder

der Zustände führt, beispielsweise kutanes T-Zell-Lymphom (CTCL), chronisch lymphozytische Leukämie (CLL), Skleroderma, systemischer Lupus Erythematosus (SLE), Psoriasis, Pemphigus psoriatische Arthritis, atopische Dermatitis, adulte T-Zell-Leukämie (ATL), AIDS (ARC), rheumatoide Arthritis, Multiple Sklerose (MS), und Abstoßungsreaktionen bei Herztransplantaten und bei Organtransplantaten wie Niere, Leber,. Mit Patienten sind menschliche Patienten gemeint.

Das erfindungsgemäße Reagens enthält das gewünschte Psoralen, gelöst in Alkohol, Propylenglykol und Wasser. Psoralenlösungen im pH-Bereich von 2-6 sind bevorzugt, da Psoralene, insbesondere 8-MOP, in basischer Lösung zu Instabilität zu neigen scheinen. Jedoch können Psoralenlösungen bei pH-Werten ausserhalb dieses pH-Bereichs ebenfalls anwendbar sein. Die Lösungen können mit Puffern, wie Acetaten, Lactaten, Phosphaten, Benzoaten, Malaten, Citraten und anderen geeigneten Puffersystemen gepuffert sein, die das pH im gewünschten Bereich halten. Acetatpuffer sind bevorzugt. Die Psoralenlösung wird schliesslich mit geeigneten Säuren und/oder Basen auf das pH eingestellt. Die Lösung wird filtriert, um Sterilität sicherzustellen. Da die erfindungsgemäßen Psoralenformulierungen sehr niedrige Psoralenkonzentrationen enthalten, ist es entscheidend, dass der Gummistopfen, der das Glasfläschen für das Reagens verschliesst, so behandelt wird, dass eine Absorption des Psoralens auf oder in den Stopfen oder ein Herauslösen von Komponenten aus dem Stopfen in die Lösung vermieden wird. Empfehlenswert sind Gummistopfen mit einem undurchlässigen Filmüberzug. Bevorzugt sind Butylgummistopfen, die mit einem Fluorharz (Teflon) überzogen sind.

Das erfindungsgemäße sterile Psoralen-Reagens kann auch in lyophilisierter Form vorliegen. Die Lyophilisierung ist ein ausgezeichnetes Mittel, eine sterile Dosierungsform zu erhalten, die über lange Zeiträume aufbewahrt werden kann. Nach Rekonstitution mit einem geeigneten Lösungsmittel ist die Psoralenlösung dann gebrauchsfertig. In den erfindungsgemäßen lyophilisierten Formulierungen sind Stoffe anwesend, die als Stützgitter bezeichnet werden, bekannt für solche Zwecke sind Mannit, Natriumchlorid oder Dextrose. Die Lösungen werden mit akzeptablen Säuren oder Basen auf das gewünschte pH eingestellt, danach mit Wasser aufgefüllt, sterilfiltriert, in Glasfläschchen abgefüllt, in üblicher Weise lyophilisiert, verschlossen und versiegelt.

Die Psoralenkonzentration in der erfindungsgemäßen Lösung kann nach Wunsch variiert werden. Bei der extrakorporalen Verabreichung von Psoralen sind jedoch nur sehr geringe Mengen erforderlich, um die bevorzugten Blutkonzentrationen zu erreichen, die im Bereich von 25-500 ng/ml, insbesondere 50-100 ng/ml liegen. Die erfindungsgemässen Lösungen enthalten somit 10 μg/ml bis 1 mg/ml Psoralen, vorzugsweise 20 μg/ml.

Bevorzugte Psoralenreagenzien enthalten 0,005-0,05 mg/ml 8-MOP, 0,1-15% Aethanol und 25-400 mg/ml Propylenglykol.

Die vorliegende Erfindung betrifft weiterhin Psoralenreagenzien, die eine sterile wässrige Psoralenlösung in einem Behälter enthalten. Behälter, aus denen Einheitsdosen von Psoralenen verabreicht werden, können Spritzen, Ampullen oder Glasfläschchen sein, insbesondere Glasfläschchen mit einer Lichtschutzfärbung, beispielsweise gelbgefärbte Glasfläschchen, die mit teflonisierten Stopfen verschlossen sind. Um die für die Phototherapie erwünschten Blutspiegel an Psoralen zu erreichen, enthält ein Glasfläschchen etwa 10 ml einer Lösung, die 20 μg/ml Psoralen, insbesondere 8-MOP, enthält.

8-MOP ist das bevorzugte Psoralen für das erfindungsgemäße Reagens, obwohl andere Psoralenderivate ebenfalls anwendbar sind. 8-MOP kann von natürlichen Quellen, nämlich der Frucht der Ammi Majus Linn Pflanze erhalten werden. Siehe beispielsweise Fahmy et al., "Ammi Majus Linn. Pharmacognostical Study and Isolation of Crystalline Constituent, Ammoidia", Quant. J. Pharm. and Pharmacol., 20:281 (1948). Weiterhin sind synthetische Verfahren für Psoralene im US Patent Nr. 4,130,568 beschrieben.

Im Zusammenhang mit der vorliegenden Erfindung besteht Phototherapie darin, dass den zu behandelnden Patienten Blut entnommen wird, dem extrakorporal eine wirksame Menge des erfindungsgemäßen Reagenzes zugesetzt wird. Im allgemeinen bewirken ein oder mehrere Fläschchen mit 10 ml Lösung mit 20 μg/ml oder 50 ml einer Lösung mit 20 μg/ml die nötige Blutkonzentration zur wirksamen Phototherapie. Die erfindungsgemäße Reagenzlösung kann dem Blut des Patienten sogleich nach der Entnahme zugesetzt werden, vor der Trennung des Blutes in Leukozyten-reiche und Leukozyten-arme Fraktionen, oder die Komposition kann der Leukozyten-reichen Fraktion unmittelbar vor der Bestrahlung zugesetzt werden. Vorzugsweise wird das Psoralen unmittelbar nach der Blutentnahme zugesetzt, damit mehr Zeit zur Verteilung des Psoralens im Blut zur Verfügung steht.

Die Leukozyten-reiche Fraktion des Blutes, das Psoralen enthält, wird dann mit UV-Strahlung im Wellenlängenbereich von etwa $2\,000 \cdot 10^{-10}$ m - $4\,000 \cdot 10^{-10}$ m (2'000-4'000 A,) vorzugsweise $3\,200 \cdot 10^{-10}$ m - $4\,000 \cdot 10^{-10}$ m (3'200-4'000 A) bestrahlt. Eine wirksame Psoralenmenge ist diejenige, die die Nukleinsäuren aus Lymphozyten bindet und nach der Bestrahlung in die zelluläre DNA interkaliert. Vor der UV-Bestrahlung, oder Aktivierung, haben Psoralene generell wenig oder gar keinen Effekt auf die Chemie der Zelle. Nach der Bestrahlung bildet das extrakorporal verabreichte Psoralen Komplexe mit der zellulären Nukleinsäure und inaktiviert diese, wobei eine Hemmung der metabolischen Funktion der Zelle resultiert. Zelltod tritt infolge Unfähigkeit zur Teilung ein. Die Zellen mit der intensivsten metabolischen Aktivität sind am empfänglichsten für eine

Ausschaltung durch Psoralen-Photophorese.

In den US Patenten Nrs. 4,321,919, 4,612,007, 4,683,889, 4,613,322 und 4,684,521 sind Phototherapieverfahren angegeben, in denen die erfindungsgemäße Reagenzkomposition verwendet werden kann.

Phototherapie in Verbindung mit Psoralenen ist bekanntlich zur Behebung eines breiten Bereichs von Leukozytenmediierten Krankheitsbildern wirksam, vgl. New England Journal of Medicine, 316;297 (1987) und Scientific American, August 1988, S. 68. Beispielsweise kann CTCL, ein Lymphozyten-mediierter Krankheitszustand, der sich in Hautschäden manifestiert, wirksam mit 8-MOP/UV-A-Photophoresetherapie behandelt werden. Das erfindungsgemäße Psoralen-Reagens (vorzugsweise 8-MOP) kann dem Patientenblut unmittelbar nach der Abnahme und vor der Trennung des Blutes in Leukozytenreiche und Leukozyten-arme Fraktionen zugesetzt werden. Dadurch steht mehr Zeit zur Verfügung, dass sich das 8-MOP unter den Blutbestandteilen verteilt. Das Blut wird dann in Leukozyten-reiche oder Leukozyten-arme Fraktionen aufgetrennt und die Leukozyten-reiche Fraktion wird durch UV-A-Bestrahlung photoaktiviert. Diese Photoaktivierung bewirkt, dass Zellen, die 8-MOP absorbiert haben, Vernetzungen zwischen den Stickstoffbasen benachbarter Stränge der DNA-Helix ausbilden. Diese Vernetzung vermindert die Replikation abnormaler Lymphozyten-Populationen. Blutbestandteile ohne Zellkerne, wie rote Blutzellen, enthalten keine DNA und werden daher nicht beeinflusst. Die der Bestrahlung ausgesetzte Blutfraktion, die die Lymphozyten enthält, die durch photoaktiviertes Quervernetzen neutralisiert sind, werden in den Patienten zurückgeleitet. Tierstudien deuten darauf hin, dass die Rückführung dieser vernetzten Zellen eine Immunantwort stimuliert und das Wachstum spezifischer Lymphozyten unter Populationen wesentlich vermindert. Die Häufigkeit der Behandlung kann von den Bedürfnissen des Patienten in grösserem Masse abhängen. In einigen Fällen kann eine Therapie dreimal wöchentlich notwendig sein, wogegen in anderen Fällen zwei Therapien alle 5 Wochen ausreichen können. In einem typischen Behandlungsschema erhielten Patienten Photophoresebehandlungen jede 5. Woche an zwei aufeinanderfolgenden Tagen.

Andere Leukozyten-mediierte Zustände, die auf Photophorese mit dem erfindungsgemäßen Reagens ansprechen, sind CLL, Scleroderma, SLE, Psoriasis, Pemphigus, psoriatische Arthritis, atopische Dermatitits, ATL, AIDS (ARC), rheumatoide Arthritis, MS, Abstoßung von Herztransplantaten oder Organtransplantaten, wie Niere und Leber. In diesen Fällen wird das erfindungsgemäße Reagens im Patientenblut extrakorporal verabreicht. Im allgemeinen ist es erforderlich, dass die Blutpsoralenkonzentration 50-100 ng/ml beträgt, um eine wirksame Behandlung zu gewährleisten. Weitere Bereiche wie 25-500 ng/ml sind jedoch akzeptabel.

Die nachstehenden Beispiele erläutern die Erfindung weiter.

Beispiel 1

Herstellung einer 8-MOP-Lösung mit 20 µg/ml

| Bestandteile | | pro ml |
|---|---|---|
| 8-MOP | | 0,02 mg |
| Aethanol, rein | | 0,05 ml |
| Propylenglykol | | 50,00 mg |
| Wasser für Injektionszwecke | q.s. | 1,00 ml |

Das 8-MOP wird in Alkohol gelöst, worauf das Propylenglykol zugegeben wird. Danach wird das Wasser zugesetzt und bis zur Homogenität gemischt. Die Lösung wird durch ein Bakterienfilter steril filtriert, die sterile Lösung in Glasfläschchen abgefüllt, verschlossen und sterilisiert.

Beispiel 2

Herstellung von Acetat-gepufferten 8-MOP-Lösungen mit 20 µg/ml

Diese Lösungen wurden mit Puffern im pH-Bereich 3,5-5,5 hergestellt, da sich im Labor gezeigt hatte, dass 8-MOP in sauren Lösungen stabiler ist. Insbesondere wurden Lösungen vom pH 3,5, 4,5, 5,0 und 5,5 hergestellt.

Formulierung

| Inhaltsstoffe | | per ml |
|---|---|---|
| 8-MOP | | 0,02 mg |
| Aethanol, rein | | 0,05 ml |
| Propylenglykol | | 50,00 mg |
| Essigsäure | | 0,0012 ml |
| Natriumhydroxidlösung, 10% Gew./Vol.* | | q.s. pH |
| Natriumacetat** | | q.s. pH |
| Wasser für Injektionszwecke | q.s. | 1,00 ml |

* Natriumhydroxidlösung zur Einstellung von pH 5,0 oder 5,5.

** Natriumacetat zur Einstellung auf pH 3,5 oder 4,5.

N.B. Natriumhydroxid und Natriumacetat werden nicht gleichzeitig in derselben Formulierung verwendet.

Herstellungsverfahren

1. 8-MOP wird in Alkohol gelöst.
2. Propylenglykol wird zugemischt.
3. Die Lösung wird mit Wasser für Injektionszwecke auf etwa 95% des gewünschten Endvolumens gebracht.
4. Essigsäure wird zugemischt.
5. Natriumacetat oder Natriumhydroxidlösung wird bis zum gewünschten pH zugesetzt.
6. Die Lösung wird mit Wasser für Injektionszwecke auf das gewünschte Endvolumen gebracht.
7. Die Lösung wird durch ein Bakterienfilter in ein steriles Glasfläschchen filtriert.
8. Das Glasfläschchen wird aseptisch gefüllt, verschlossen und schließlich sterilisiert.

Beispiel 3

Herstellung von 8-MOP 200 µg/Fläschchen als lyophilisiertes Pulver

Bei dieser Formulierung wurde als Stützgitter Mannit verwendet, obgleich andere Substanzen, die für Lyophilisierungszwecke bekannt sind, wie Natriumchlorid und Dextrose, ebenfalls verwendet werden können. Das lyophilisierte Pulver kann mit 10 ml eines Lösungsmittels aus Wasser, Aethanol und Propylenglykol in eine Lösung mit einer Konzentration von 20 µg/ml umgewandelt werden.

Formulierung

| Inhaltsstoffe | | pro 5 ml hergestellte Lösung | im Lyophilisat pro Fläschchen |
|---|---|---|---|
| 8-MOP | | 0,20 mg | 0,20 mg |
| Mannit | | 50,00 mg | 50,00 mg |
| Essigsäure | | 0,012 ml | Spuren |
| Natriumhydroxidlösung; 10% (Gew./Vol.) | q.s. | pH 3,5 | Spuren |
| Wasser für Injektionszwecke | q.s | 5,00 ml | * |

* praktisch vollständig verflüchtigt während der Lyophilisierung.

Herstellungsverfahren

1. 90-95% des Wassers für Injektionszwecke werden etwa auf 95°C erwärmt.
2. 8-MOP wird unter Rühren gelöst.
3. Die Lösung wird auf Raumtemperatur gekühlt.
4. Mannit wird zugegeben und gelöst.
5. Essigsäure wird zugemischt.
6. Natriumhydroxidlösung wird bis zu einem pH 3,5* zugesetzt.
7. Wasser für Injektionszwecke wird bis zum Endvolumen zugesetzt.
8. Die Lösung wird durch ein Bakterienfilter in einen sterilen Behälter filtriert.
9. Der Behälter wird aseptisch gefüllt, mit Lyophilisierungsstopfen partiell verschlossen und lyophilisiert.
10. Stopfen und Fläschchen werden versiegelt.

* Dieses pH wurde beim vorliegenden Experiment eingestellt, jedoch können andere saure pH-Werte zwischen 3.5 und 5.5 eingestellt werden.

## Patentansprüche

1. Psoralen-Reagenz zur Anwendung bei der extrakorporalen Behandlung von Blut bei UV-A-Photophorese enthaltend eine sterile, wässrige Lösung von 0,01 - 1 mg/ml 8-Methoxypsoralen (8-MOP) , O,1-15 % Aethanol, 25-400 mg/ml Propylenglykol und ein Puffersystem zur Aufrechterhaltung eines pH 3,5-5,5.

2. Reagenz gemäß Anspruch 1, enthaltend 0,005-0,5 mg/ml 8-MOP.

3. Reagenz gemäß Anspruch 2 in einem gefärbten Glasfläschen, das mit einem Fluor-Harz-laminierten (teflonisierten) Gummistopfen verschlossen ist.

4. Reagenz gemäß Anspruch 3 in einem gefärbten Glasfläschen, das mit einem Fluor-Harz-laminierten (teflonisierten) Butylgummistopfen verschlossen ist.

5. Reagenz gemäß den Ansprüchen 4 zur Anwendung bei der extrakorporalen Behandlung von Blut bei UV-A-Photophorese zwecks Bekämpfung Leukozyten-mediierter Krankheiten.

6. Reagenz gemäß den Ansprüchen 1-4 zur Anwendung gemäss Anspruch 6, wobei die Leukozyten-mediierte Krankheit kutanes T-Zell-Lymphom (CTCL), chronisch lymphozytische Leukämie (CLL), Skleroderma, systemischer Lupus Erythematosus (SLE), Psoriasis, Pemphigus, psoriatische Arthritis, atopische Dermatitits, adulte T-Zell-Leukämie (ATL), AIDS (ARC), rheumatoide Arthritis, Multiple Sklerose (MS) oder Abstoßungsreaktionen bei Herztransplantaten und bei Organtransplantaten sind.

## Claims

1. A psoralen reagent for use in the extracorporeal treatment of blood in UV-A photophoresis, containing a sterile, aqueous solution of 0.01 -1 mg/ml 8-methoxypsoralen (8-MOP), 0.1-15% ethanol, 25-400 mg/ml propylene glycol and a buffer system for the maintenance of a pH of 3.5-5.5.

2. A reagent in accordance with claim 1, containing 0.005-0.5 mg/ml 8-MOP.

3. A reagent in accordance with claim 2 in a coloured glass vial which is closed with a fluoro-resin-laminated (teflonized) rubber stopper.

4. A reagent in accordance with claim 3 in a coloured glass vial which is closed with a fluoro-resin-laminated (teflonized) butyl rubber stopper.

5. A reagent in accordance with claims 1-4 for use in the extracorporeal treatment of blood in UV-A photophoresis for the control of leukocyte-mediated conditions.

6. A reagent in accordance with claims 1-4 for use in accordance with claim 6, wherein the leukocyte-mediated condition is cutaneous T-cell lymphoma (CTCL), chronic lymphocytic leukemia (CLL), scleroderma, systemic lupus erythematosis (SLE), psoriasis, pemphigus, psoriatic arthritis, atopic dermatitis, adult T-cell leukemia (ATL), AIDS (ARC), rheumatoid arthritis, multiple sclerosis (MS) or rejection reactions in cardiac transplants and in organ transplants.

## Revendications

1. Réactif à base de psoralène pour utilisation dans le traitement extracorporel de sang avec photorèse aux UV-A, contenant une solution aqueuse stérile de 0,01-1 mg/ml de 8-méthoxypsoralène (8-MOP), 0,1-15 % d'éthanol, 25-400 mg/ml de propylèneglycol et un système tampon pour le maintien d'un pH de 3,5-5,5.

2. Réactif selon la revendication 1, contenant 0,005-0,5 mg/ml de 8-MOP.

3. Réactif selon la revendication 2, dans un flacon de verre teinté, qui est bouché avec un bouchon de caoutchouc (au Téflon) stratifié à base de résine fluorée.

4. Réactif selon la revendication 3, dans un flacon de verre teinté, qui est bouché avec un bouchon de caoutchouc butyle (au Téflon) stratifié à base de résine fluorée.

5. Réactif selon les revendications 1-4, pour utilisation dans le traitement extracorporel de sang avec photorèse aux UV-A pour combattre des maladies médiées par des leucocytes.

6. Réactif selon les revendications 1-4, pour utilisation selon la revendication 6, les maladies médiées par des leucocytes étant le lymphome cutané à cellules T (LCCT), la leucémie lymphocytaire chronique (LLC) la sclérodermie, le lupus érythémateux disséminé (LED), le psoriasis, le pemphigus, l'arthropathie psoriasique, la dermatite atopique, la leucémie à cellules T de l'adulte (LTA), le SIDA (l'ARC), la polyarthrite rhumatoïde, la sclérose en plaques (SP) ou des réactions de rejet dans des greffes du coeur et des greffes d'organes.